# EUROPÄISCHE PATENTSCHRIFT

(11) **EP 0 984 913 B1**
(45) Veröffentlichungstag und Bekanntmachung des Hinweises auf die Patenterteilung: **21.11.2001**
(21) Anmeldenummer: 98925534.4
(22) Anmeldetag: 05.05.1998
(51) Int. Cl.: C07C 37/20, C07C 37/74, C07C 37/82, C07C 37/84, C07C 39/16

(54) **VERFAHREN ZUR KONTINUIERLICHEN HERSTELLUNG VON DIHYDROXYDIARYLALKANEN**
METHOD FOR CONTINUOUS PRODUCTION OF DIHYDROXYDIARYLALKANES
PROCEDE DE PRODUCTION EN CONTINU DE DIHYDROXYDIARYLALCANES

(30) Priorität: 16.05.1997 DE 19720539
(43) Veröffentlichungstag der Anmeldung: 15.03.2000
(73) Patentinhaber: Bayer Aktiengesellschaft, 51368 Leverkusen (DE)
(72) Erfinder: FENNHOFF, Gerhard, D-47877 Willich (DE); BUYSCH, Hans-Josef, D-47809 Krefeld (DE); FENGLER, Gerd, D-47802 Krefeld (DE)
(86) Internationale Anmeldenummer: EP9802643
(87) Internationale Veröffentlichungsnummer: WO9852895

(56) Entgegenhaltungen:
- EP-A- 0 332 203
- WO-A-92/09550
- US-A- 4 308 404
- US-A- 4 391 997
- PATENT ABSTRACTS OF JAPAN vol. 009, no. 166 (C-290), 11. Juli 1985 & JP 60 038335 A (MITSUI TOATSU KAGAKU KK), 27. Februar 1985
- DATABASE WPI Section Ch, Week 9349 Derwent Publications Ltd., London, GB; Class A41, AN 93-392597 XP002075636 & JP 05 294 873 A (CHIYODA CORP)

## Beschreibung

Vorliegende Erfindung betrifft ein Verfahren zur kontinuierlichen Herstellung von Dihydroxydiarylalkanen (Bisphenolen) durch Umsetzung von frischem Phenol, Phenol und Isoalkenylphenol aus der Spaltung von Beiprodukten und von Keton. Dabei gewinnt man aus dem Reaktionsgemisch die Hauptmenge Bisphenol durch Kristallisation, befreit die dabei erhaltene Mutterlauge destillativ von Phenol. Das Phenol führt man wieder in die Reaktion, den bei der Destillation erhaltenen Sumpf spaltet man nach Zugabe eines basischen Katalysators in einer Reaktivrektifikation. Das über Kopf gehende Phenol und Isoalkenylphenol wird in die Reaktion zurückgeleitet, der Sumpf aus der 1. Reaktivkolonne sauer gestellt und in Gegenwart eines sauren Katalysators in einer 2. Reaktivrektifikation weiter gespalten in abdestillierendes Phenol, das in der Reaktion wiederverwendet, und einen Sumpf, der entsorgt wird.

Es ist bekannt, Bisphenole herzustellen durch sauer katalysierte Umsetzung von Ketonen mit Phenol. Dazu gibt es eine Reihe unterschiedlicher Vorschläge (vergl. z.B. US-A 2 775 620, EP-A 342 758, EP-A 616 993, DE-OS 3 833 900, US-A 4 308 404, US-A 4 308 405, EP-A 630 878, US-A 4 876 391, US-A 3 242 219). US-A 4391997 offenbart ein Verfahren zur Herstellung von Bisphenol-A in einer Reaktorserie mit ansteigendem Temperaturgradienten.

In Ullmann's Encyclopedia of Indust. Chem., 5. Ed., Vol. A 19, p. 348-52 findet sich eine Übersicht über die ältere Literatur zur Bisphenolherstellung. In der Regel werden Bisphenole, besonders Bisphenol A (BPA), das technisch wichtigste der Bisphenole so hergestellt, daß man Keton und Phenol zu einer im Kreis geführten Mutterlauge aus der Bisphenol-Aufarbeitung hinzusetzt, dieses Gemisch über saure Ionenaustauscher leitet und die Umsetzung zu Bisphenol vollzieht. Aus dem Reaktionsgemisch wird gegebenenfalls nicht umgesetztes Keton zurückgewonnen und in die Reaktion zurückgeleitet. Das Reaktionsgemisch wird abgekühlt, das Bisphenol läßt man gegebenenfalls als Phenoladdukt auskristallisieren, trennt es ab und wäscht mit Phenol. Aus dem Addukt wird durch Flashdestillation das Phenol abgetrennt und reines Bisphenol gewonnen. Die Mutterlauge der Kristallisation wird über saure Ionenaustauscher geleitet und darin befindliche Isomere zu Bisphenol umgelagert. Das so entstandene Bisphenol wird per Kristallisation abgetrennt und das erhaltene Kristallisat in den ersten Kristallisationsschritt eingeschleust. Aus der nun erhaltenen Mutterlauge zweigt man einen Teil (ca. 10 bis 20 %) ab, die Hauptmenge wird in die Reaktion zurückgeführt. Aus dem abgezweigten Teil wird Phenol abdestilliert und in die Reaktion zurückgeführt. Den bei der Destillation erhaltenen Rückstand entfernt man aus den Verfahren aus und verwendet ihn z.B. für die Herstellung von Phenolharz.

Bei dieser Verfahrensweise, die relativ kompliziert ist, wird die Mutterlauge immer wieder über den Katalysator geleitet. Dabei entstehen viele Isomere und Nebenprodukte und auch gefärbte Verbindungen, die sorgfältig von Bisphenol abgetrennt werden müssen, was gegebenenfalls eine weitere Kristallisation nötig macht. Zum anderen verliert man eine nennenswerte Menge an wertvollen Verbindungen wie Bisphenol und Isomere mit dem abgezweigten und schließlich ausgeschleusten Teil. Diese Entnahme aus der Mutterlauge ist unbedingt erforderlich, um die Menge an unbrauchbaren und störenden Verbindungen nicht zu groß werden zu lassen.

Man hat daher versucht, den ausgeschleusten Anteil aufzuarbeiten und zu spalten, um die Wertstoffe für die Bisphenolherstellung zurückzugewinnen. Auch dazu gibt es eine Reihe von Vorschlägen. Man kann durch Pyrolyse bei ca. 300° Phenol und Alkenylphenole, die noch gründlich gereinigt werden müssen, in mäßiger Ausbeute erhalten (US-A 2 497 503). Auch eine hydrierende Behandlung führt zu Wertprodukten, wie EP-A 17 852 lehrt. Die Spaltung kann auch durch saure und basische Verbindungen beschleunigt werden. Mit Säuren wie Schwefel- oder Toluolsulfonsäure erhält man allerdings nur Phenol (US-A 3 466 337). Basische Katalysatoren dagegen bewirken eine Spaltung der ausgeschleusten Materialien in Phenol und Isoalkenylphenol. Als Katalysatoren werden genannt Alkaliverbindungen wie NaOH, KOH, NaHCO₃, Na-Acetat, Na-Hypophosphit, K₂CO₃, MgO und Al-Isopropylat (US-A 4 277 628, US-A 4 594 459, US-A 4 131 749). Bei dieser Vorgehensweise wird allerdings nur ein Teil des ausgeschleusten Materials gespalten, und man arbeitet diskontinuierlich oder halbkontinuierlich Vollkontinuierliche Verfahren sind unbekannt.

Diese Verfahren sind im Hinblick auf eine höhere Reinheit des Bisphenols dahingehend verbessert worden, daß man das Phenol/Isoalkenylphenol-Gemisch aus der Spaltung in die erste Mutterlauge nach Abtrennung der ersten Partie Bisphenol einspeist. Man leitet das Gemisch über den sauren Katalysator und läßt die Umsetzung von Isoalkenylphenol mit Phenol und die Umlagerung gleichzeitig ablaufen. Aus der Mischung wird dann die zweite Partie Bisphenol durch Kristallisation abgetrennt und die zweite Mutterlauge wieder zur Spaltung geführt. Die zweite Bisphenol-Partie wird in die erste Kristallisation gegeben. Dadurch wird zwar die Reinheit des Bisphenols etwas erhöht, aber man kompliziert das Verfahren um einen weiteren Kreislauf (US-A 4 954 661).

Vorgeschlagen wurde auch, die zweite Mutterlauge im oben angegebenen Bisphenolprozeß oder die erste Mutterlauge nach Abtrennen der ersten Bisphenolmenge und nach der Umlagerung zumindestens teilweise destillativ aufzuarbeiten, um die darin enthaltenen Wertprodukte besser zu nutzen. Das dabei erhaltene Bisphenol wird in die erste Kristallisationsstufe zurückgegeben und dort gereinigt. Die Isomeren enthaltenden Leichtsieder führt man der Reaktion zu (WO 94/20445 und EP-A 552 518). Die Schwierigkeit besteht darin, aus der an Isomeren und Nebenprodukten stark angereicherten Mutterlauge mit vertretbarem Aufwand hinreichend reine Fraktionen zu erhalten, die besonders arm an den schwer abtennbaren Chromanen und Indanen sind, ohne daß man den nicht mehr brauchbaren Rest zu sehr vergrößert und die Ausbeute schmälert. Deswegen muß ein Teil der Destillationsprodukte verworfen werden.

Die bei der Destillation erhaltenen Bisphenol-armen Fraktionen kann man auch der Spaltung unterwerfen und die Spaltprodukte in das Verfahren wieder einführen (EP-A 332 203).

Es wurde nun gefunden, daß man eine hervorragende Ausbeute an Bisphenol großer Reinheit nach einem einfachen vollkontinuierlichen Verfahren erhält, wenn man Phenol mit Keton und Isoalkenylphenol aus der Spaltung so umsetzt, daß möglichst wenig Isomere und Nebenprodukte entstehen, aus dem Reaktionsgemisch nicht umgesetztes Keton abdestilliert, gegebenenfalls saure Bestandteile abtrennt, die Hauptmenge des darin enthaltenen Bisphenols durch Kristallisation isoliert, die Mutterlauge und das Waschphenol aus diesem Kristallisationsschritt vereinigt, Phenol abdestilliert und in die Reaktion gibt, den Sumpf mit einem basischen Katalysator vermischt und in einer ersten Reaktivrektifikation in über Kopf gehendes Phenol und Isoalkenylphenol spaltet, die beide in die Reaktion fließen, den bei der Reaktivrektifikation erhaltenen Sumpf sauer stellt und in Gegenwart eines sauren Katalysators in einer zweiten Reaktivrektifikation in Phenol, das der Reaktion zugegeben wird, und einen zu entsorgenden Rückstand spaltet.

Gegenstand der Erfindung ist somit das im Patentanspruch gekennzeichnet Verfahren.

Geeignete aromatische Hydroxyverbindungen für das erfindungsgemäße Verfahren sind in p-Position nicht substituiert und enthalten keine Substituenten zweiter Ordnung wie Cyano-, Carboxy- oder Nitrogruppen; genannt seien beispielsweise Phenol, o- und m-Kresol, 2,6-Dimethylphenol, o-tert.-Butylphenol, 2-Methyl-6-tert.-butylphenol, o-Cyclohexylphenol, o-Phenylphenol, o-Isopropylphenol, 2-Methyl-6-cyclopentyl-phenol, o- und m-Chlorphenol, 2,3,6-Trimethylphenol. Bevorzugt sind Phenol, o- und m-Kresol, 2,6-Dimethylphenol, o-tert.-Butylphenol und o-Phenyl-phenol; besonders bevorzugt ist Phenol.

Geeignete Ketone enthalten wenigstens eine aliphatische Gruppe an der Carbonylfunktion; genannt seien beispielsweise Aceton, Methylethylketon, Methylpropylketon, Methylisopropylketon, Diethylketon, Acetophenon, Cyclohexanon, Cyclopentanon, Methyl-, Dimethyl- und Trimethylcyclohexanone, die auch geminale Methylgruppen aufweisen können wie 3,3-Dimethyl-5-methylcyclohexanon (Hydroisophoron). Bevorzugt sind Aceton, Acetophenon, Cyclohexanon und dessen Methylgruppen tragende Homologe; besonders bevorzugt ist Aceton.

Geeignete Katalysatoren für die basische Spaltung sind die in der Literatur genannten, bevorzugt Alkalioxide und -hydroxide, besonders bevorzugt NaOH und KOH. Sie werden im erfindungsgemäßen Verfahren in die Schmelze der Spaltungsedukte eingetragen, gelöst und homogen verteilt, wobei die Temperatur zweckmäßig zwischen 100 und 200°C, bevorzugt 120 und 180°C liegt.

Geeignete Katalysatoren für die saure Spaltung sind die in der Literatur angeführten, bevorzugt Schwefelsäure, Phosphorsäure, phosphorige Säure, deren partielle Salze wie NH₄HSO₄, NaHSO₄, KHSO₄, NaH₂PO₄, KH₂PO₄, NH₄H₂PO₄, NH₄H₂PO₃, KH₂PO₃ und Analoga, ferner die organischen Abkömmlinge dieser Säuren nämlich aromatische Sulfonsäuren und Disulfonsäuren wie Benzol-, Toluol-, Xylol-, Phenolsulfonsäure, Diphenyldisulfonsäure, Diphenyletherdisulfonsäure, sodann aromatische Phosphon- und Phosphinsäuren wie Benzol-, Toluol-, Xylolphosphon-und -phosphinsäure, Diphenyl-diphosphon- und diphosphinsäure, ferner feste Säuren wie saure Aluminiumoxide, Tonerden wie Bentonite und Montmorillonite, Zeolithe, Titan- und Zirkonoxide, Niob- und Tantaloxide; bevorzugt sind saure Tonerden.

Die Mengen an Katalysatoren, die dem zu spaltenden Gemisch zugegeben werden, liegen zwischen 0,01 Gew.-% und 5 Gew.-%, bevorzugt 0,05 bis 3 Gew.-%, besonders bevorzugt 0,1 bis 2 Gew.-%, bezogen auf die Menge an Spaltgemisch. Um diese Mengenregel für saure Katalysatoren einhalten zu können, müssen die basischen Katalysatoren nach der basischen Spaltung entweder aus dem Sumpf entfernt werden, was in aller Regel zu aufwendig sein dürfte, oder sie müssen zuerst neutralisiert werden durch eine starke Säure, bevor der Zusatz der erforderlichen Menge an saurem Katalysator erfolgt. Auf diese Weise ist sichergestellt, daß hinreichende Mengen aktiver Säure vorliegen. Sauerstellen im Sinne der Erfindung bedeutet also, daß man dem Sumpf der basischen Spaltung soviel an Säure zugibt, daß sowohl der darin befindliche basische Katalysator neutralisiert wird als auch darüber hinaus eine hinreichende Menge an saurem Katalysator für die folgende saure Spaltung zur Verfügung steht.

Die Katalysatoren können beispielsweise gelöst oder suspendiert in Phenol kontinuierlich dem in der Spaltkolonne fließenden Strom zudosiert werden. Es ist jedoch auch möglich, sie in Zwischenbehältern diskontinuierlich dem zu spaltenden Material beizumischen und dann dieses Gemisch kontinuierlich in die Spaltkolonne zu fördern.

Die Kolonnen, in denen die Reaktivrektifikationen durchgeführt werden, entsprechen im allgemeinen üblichen Destillationskolonnen. In den Kolonnen ist der Spaltung eine destillative und fraktionierende Trennung der Spaltprodukte Phenol und Isoalkenylphenol von den nicht gespaltenen oder auch nicht spaltbaren Verbindungen überlagert. Die Spaltprodukte gehen dabei, im allgemeinen schon rein genug für ihre weitere Verwendung, über Kopf. Die nicht spaltbaren Anteile werden als Sumpf ausgetragen. Reaktivrektifikationen sind dem Fachmann bekannt und beispielsweise in Ullmann's Encyclopedia of Industrial Chemistry, 5. Ed., Vol. B 4, p. 321-8 beschrieben.

Im vorliegenden Fall kann es vorteilhaft sein, in den Spaltkolonnen entsprechend dem während der Spaltung von oben nach unten in der Kolonne abnehmenden Flüssigkeits-und Gasvolumen den Kolonnendurchmesser im unteren Teil angemessen abnehmen zu lassen. Für die Einstellung einer geeigneten Verweilzeit des Spaltmaterials in der Kolonne ist es zweckmäßig, Böden in die Spaltkolonnen einzuziehen. Dies gilt besonders für den Teil, in dem die Spaltung abläuft. Im oberen Teil kann für eine effektive Trennung der Produkte die Verwendung von Füllkörpern oder Packungen angezeigt sein. Für den Fall, daß die Reinheit des aus der zweiten Spaltkolonne abdestillierenden Phenols für die Verwendung in der Bisphenolsynthese nicht ausreichend ist, kann man es in eine weitere Kolonne oder aber in die erste Spaltkolonne einführen und dort weiter reinigen.

Bevorzugte Verfahrensweisen für die Umsetzung zum Bisphenol sind solche, die möglichst hochselektiv Bisphenol liefern. Sie zeichnen sich dadurch aus, daß man bei möglichst niedriger Ketonkonzentration arbeitet. Dies läßt sich erreichen, indem man Phenol mit Keton und Isoalkenylphenol in mindestens zwei in Reihe geschalteten Reaktoren umsetzt, die saure Ionenaustauscher enthalten und bei möglichst niedriger Temperatur, aber in Richtung fortschreitender Umsetzung mit steigenden Temperaturen betrieben werden, wobei die Gesamtmenge an Keton und Isoalkenylphenol auf die einzelnen Reaktoren aufgeteilt und vor dem Eintreten in die jeweiligen Reaktoren homogen im Reaktionsgemisch verteilt wird. Die Selektivität für Bisphenol kann noch erhöht werden, wenn man die Verteilung von Keton und Isoalkenylphenol so steuert, daß der Anteil der Gesamtmenge pro Reaktor umso kleiner wird, je höher die Temperatur des betreffenden Reaktors liegt und die Reaktorkaskade innerhalb einer Temperaturspanne von max. 35 bis 85°C, vorzugsweise 38 bis 75°C, betrieben wird.

Vor den einzelnen Reaktoren sind zweckmäßig sowohl Mischorgane bekannter Bauart angebracht, um die homogene Verteilung von Keton und Isoalkenylphenol im Ausgangsphenol bzw. Reaktionsgemisch zu gewährleisten als auch Wärmetauscher zur gewünschten Temperierung des Gemisches vor dem Durchgang durch das Katalysatorbett.

Nach dem Verlassen des letzten Reaktors der Kaskade und Abtrennung von nicht umgesetztem Keton wird das Reaktionsprodukt gegebenenfalls von sauren Bestandteilen befreit, wie dies beispielsweise in US-A4 876 391 oder EP-A 552 518 beschrieben ist und gegebenenfalls einer Feinfiltration zur Abtrennung von Katalysator- und Apparateabrieb oder sonstigen festen Verunreinigung unterzogen. Dann fließt das Reaktionsgemisch in einen für die Bisphenol-Synthese üblichen, dem Fachmann bekannten Kristaller (Ullmann's Encyclopedia of Industrial Chemistry, 5. Ed., Vol. B 2, p. 3-1 bis 3-34; ein Kristallisierverfahren findet sich z.B. in US 4 209 646), in dem die Hauptmenge des darin enthaltenen Bisphenols auskristallisiert (im Falle von Bisphenol A als Addukt mit Phenol), durch Filtration etwa mit einem Drehfilter abgetrennt und mit Phenol gewaschen wird. Aus der mit dem Waschphenol vereinigten Mutterlauge wird das Reaktionswasser und etwas Phenol abdestilliert, daraus das Phenol wie üblich durch Extraktion abgetrennt. Das Wasser wird entsorgt, das Phenol in die Reaktion zurückgeführt. Aus der Mutterlauge wird nun Phenol vollständig im Vakuum abdestilliert. Der anfallende Sumpf wird mit einem basischen Katalysator vermischt, kontinuierlich in eine Reaktivrektifikation eindosiert und darin bei 190 bis 270°C, bevorzugt 200 bis 260°C, besonders bevorzugt 210 bis 250°C und 15 bis 0,5 mbar, bevorzugt 12 bis 1 mbar, besonders bevorzugt 10 bis 1 mbar, gespalten. Das Destillat, bestehend aus Phenol und Isoalkenylphenol und dessen Oligomeren, wird der Reaktion zugeleitet. Der Sumpf wird nach Sauerstellen in eine zweite, der vorgenannten ähnlichen Reaktivrektifikation eindosiert und darin bei 150 bis 260°C, bevorzugt 160 bis 250°C, besonders bevorzugt 170 bis 240°C und den oben angegebenen Drücken gespalten, wobei Phenol abdestilliert. Dieses kann gegebenenfalls über eine Kolonne weiter gereinigt werden und wird dem Reaktionsstrom wieder beigemischt. Am Fuß der Kolonne wird ein Sumpf entnommen, der zu entsorgen ist.

Wenn der Sumpfanteil aus der basischen Spaltung aufgrund eines relativ kleinen Produktionsstranges einer Bisphenol-Anlage für den ökonomischen Betrieb einer Kolonne zu klein ist, empfiehlt sich eine Zusammenführung solcher Kleinmengen aus mehreren Strängen oder eine Sammlung des Sumpfes aus einer Anlage zu einer größeren Menge und dann deren Spaltung in einer geeigneten Apparatur.

Nach dem beschriebenen erfindungsgemäßen Verfahren lassen sich - nimmt man das unvermeidlich entstehende Reaktionswasser von Beginn an aus der Stoffbilanz heraus - Materialausbeuten bis zu 98 % erhalten; zudem erhält man ein besonders reines Bisphenol.

Die die Reaktorkaskade verlassenden Reaktionsgemische können vor der Kristallisation 10 bis 35 Gew.-%, bevorzugt 12 bis 32, besonders bevorzugt 14 bis 30 Gew.-%, Bisphenol enthalten. Sollte der Gehalt für eine Kristallisation ungünstig sein, so kann er durch Zusetzen oder Abdestillieren von Phenol angepaßt werden.

Das erfindungsgemäße Verfahren hat demnach gegenüber den bekannten Bisphenol-verfahren die Vorteile, ein sehr reines Bisphenol zu liefern mit hervorragender Ausbeute bei deutlich einfacherer und vollkontinuierlicher Durchführung.

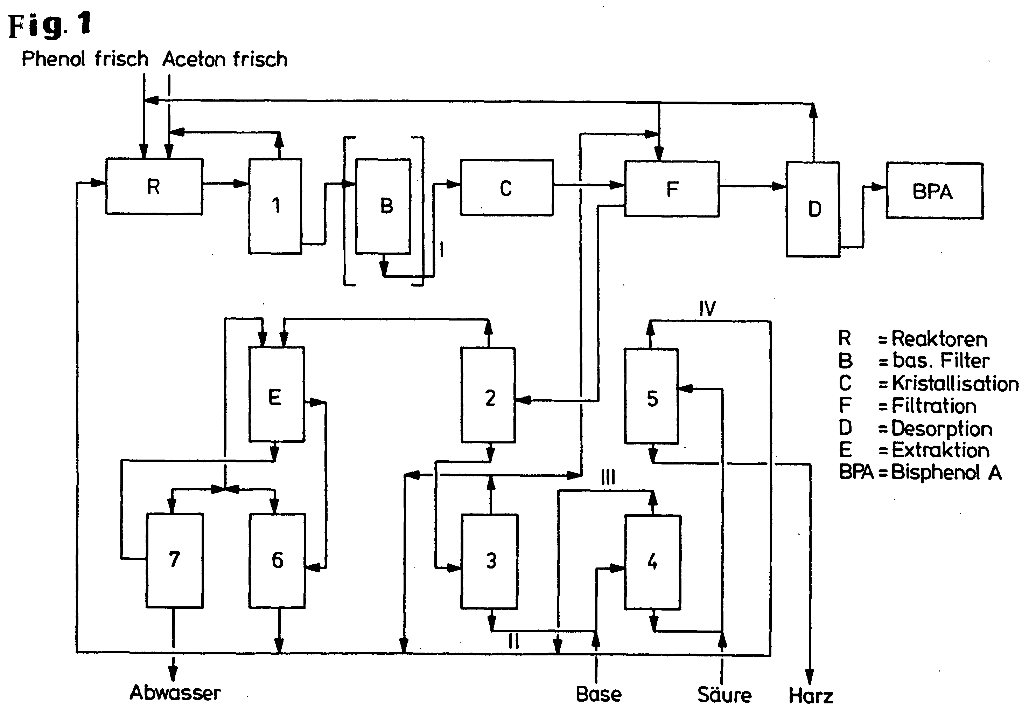

### Beispiel (vgl. Abb. 1)

In einer Kaskade von drei Reaktoren, die mit saurem Ionenaustauscher (SC 102, Bayer AG) gefüllt waren, der als Cokatalysator 3,6 Gew.-% Dimethylthiazolidin enthielt, wurde nach folgender Arbeitsweise BPA hergestellt:

In den ersten Reaktor förderte man 5438 Gew.-Teile/h Phenol zusammen mit einem Drittel/h der Menge aus 274 Gew.-Teilen Frischaceton und 402 Gew.-Teilen Phenol und Isopropenylphenol enthaltendem Spaltprodukt bei 50°C mit einer Belastung von 0,6 kg/l Katalysator und Stunde. Das austretende Reaktionsprodukt wurde mit einem weiteren Drittel/h der oben angegebenen Menge aus Aceton und Spaltprodukt vermischt, auf 60°C temperiert und in den zweiten Reaktor geleitet. Nach Verlassen des zweiten Reaktors wurde das letzte Drittel/h an oben angegebener Menge aus Aceton und Spaltprodukt ins Reaktionsprodukt eingemischt, auf 70°C temperiert und damit der 3. Reaktor beschickt. Die Belastung blieb für alle Reaktoren bei 0,6 kg/l · h. Der Acetonumsatz lag bei 97 bis 98 %.

Aus dieser kontinuierlich arbeitenden Apparatur zur Herstellung von Bisphenol A (BPA) durch mit sauren Ionenaustauschern katalysierte Umsetzung von Frischphenol, Phenol aus basischer und saurer Spaltung, Aceton und Isopropenylphenol aus alkalischer Spaltung erhielt man stündlich 6114 Gew.-Teile Reaktionsmischung mit der in Tabelle 1 angegebenen Zusammensetzung I nach destillativer Abtrennung von Resten nicht umgesetzten Acetons (1) und gegebenenfalls einer Filtration durch ein Filter und basischem Ionentauscher (B) zwecks Entfernung von aus dem sauren Ionentauscher ausgelaugter Säuren.

Daraus wurde in üblicher Weise durch Kristallisation (C), Absaugen, Waschen mit Phenol und Abpressen (F) ein BPA-Phenol-Addukt erhalten, das nach Flashdestillation des Phenols im Vakuum (D) 983 Gew.-Teile/h BPA mit einer Reinheit von 99,78% ergab. Die dabei anfallende Mutterlauge und Waschphenol wurde kontinuierlich im Vakuum eingedampft (2, 3), das abdestillierte Phenol, zum Teil nach Extraktion (E) und Trennung von Wasser und Extraktionsmittel (6,7), in Reaktion und Wäsche zurückgegeben und der Hochsiederrückstand von stündlich 425 Gew.-Teile als Schmelze II, nachdem darin 0,5 Gew.-% KOH homogen verteilt wurden, in eine 20-bödige Reaktivrektifikation (4) eindosiert, die eine Kopftemperatur von 80 bis 82°C und eine Sumpftemperatur von 238°C aufwies und bei 2 mbar betrieben wurde. Aus dieser Reaktivkolonne gingen 376 Gew.-Teile/h Destillat mit der Zusammensetzung m über, die in die Reaktion zu BPA zurückflossen. Aus dem Sumpf der Kolonne wurden 48 Gew.-Teile/h Rückstand ausgetragen. Diese wurden als Schmelze mit 2 Mol Toluolsulfonsäure pro enthaltenem Mol KOH versetzt und kontinuierlich in eine ebenfalls 20-bödige Reaktivrektifikation (5) eingeleitet, die mit einer Kopftemperatur von ca. 73 bis 76°C und einer Sumpftemperatur von ca. 205°C bei einem Druck von 5 mbar betrieben wurde.

Aus dieser zweiten Reaktivkolonne gingen 55 Gew.-% des eingespeisten Materials als Destillat mit der Zusammensetzung IV über, 45 Gew.-% wurden aus dem Sumpf als zu entsorgender Abfall ausgetragen.

Bei der Produktion von 983 Gew.-Teile BPA fielen demnach 22 Gew.-Teile Abfall an, was einer Materialausbeute von 97,8 % entspricht (nach Vorwegabzug des Wassers aus der Kondensation von Aceton und Phenol).

**Tabelle 1**

| | o,o'BP | o,p'BP | Chrom. | BPA | Ind. | Trisph. | MG 402 | NP | Σ IPENP | Phenol | Gew. Tle/h |
|---|---|---|---|---|---|---|---|---|---|---|---|
| I | 0,09 | 5,06 | 0,22 | 93,72 | 0,16 | 0,60 | 0 | 0,14 | 0 | 0 | 6114 |
| II | 0,19 | 16,10 | 0,51 | 75,76 | 0,20 | 2,10 | 0 | 1,22 | 0 | 3,91 | 425 |
| III | 0,02 | 0,05 | 0,06 | 0,38 | 0,01 | 0,01 | 0 | 0,11 | 52,01 | 47,32 | 376 |
| IV | 0,01 | 0,03 | 0,20 | 0,11 | 0,13 | 0 | 0 | 0,24 | 0 | 99,3 | 26 |

## Patentansprüche

1. Verfahren zur kontinuierlichen Herstellung von Dihydroxydiarylalkanen (Bisphenolen) aus Keton, Phenol und Isoalkenylphenol durch saure Katalyse, **dadurch gekennzeichnet, daß** man
1. in einer Kaskade von mindestens zwei Reaktoren Phenol mit Keton und Isoalkenylphenol derart umsetzt, daß man bei möglichst niedriger Temperatur, aber in Richtung zunehmenden Umsatzes steigender Temperatur arbeitet und Aceton und Isoalkenylphenol auf die einzelnen Reaktoren aufteilt, wobei man die Verteilung von Keton und Isoalkenylphenol so steuert, daß der Anteil der Gesamtmenge pro Reaktor umso kleiner wird, je höher die Temperatur des betreffenden Reaktors liegt,
2. aus dem Reaktionsgemisch nicht umgesetztes Keton abdestilliert und gegebenenfalls im Reaktionsgemisch enthaltene saure Bestandteile entfernt,
3. Bisphenol aus dem Reaktionsgemisch durch Kristallisation weitgehend abtrennt,
4. die dabei erhaltene Mutterlauge mit Waschphenol vereinigt, das Phenol abdestilliert und wieder in die Reaktion zurückführt,
5. den dabei gebildeten Bisphenol, Isomere und Nebenprodukte enthaltenden Sumpf in Gegenwart einer katalytischen Menge Base in einer Reaktivrektifikation in ein über Kopf gehendes Phenol und Isoalkenylphenol und einen hochsiedenden Sumpf spaltet,
6. diesen Hochsiedersumpf sauer stellt, so daß er eine katalytische Menge an Säure enthält, ihn in einer weiteren Reaktivrektifikation in Phenol und einen zu entsorgenden Sumpf spaltet und
7. das Phenoldestillat aus 6. und das Phenol/Isoalkenylphenoldestillat aus 5. je gegebenenfalls nach einer zusätzlichen Reinigung in die Umsatzung zu Bisphenol zurückführt.

## Claims

1. A method for the continuous production of dihydroxydiarylalkanes (bisphenols) from a ketone, phenol and an isoalkenylphenol by acid catalysis, **characterised in that**
1. a phenol is reacted with a ketone and with an isoalkenylphenol in a cascade of at least two reactors and in a manner such that the lowest possible temperature is employed consistent with progressive conversion with increasing temperature, and acetone and isoalkenylphenol are distributed over the individual reactors, wherein the distribution of ketone and isoalkenylphenol is controlled so that the proportion of the total amount thereof per reactor becomes smaller the higher is the temperature of the reactor concerned,
2. unreacted ketone is removed from the reaction mixture by distillation, and any acidic constituents which are contained in the reaction mixture are removed,
3. bisphenol is substantially separated from the reaction mixture by crystallisation,
4. the mother liquor which is thus obtained is purified with washing phenol, and the phenol is distilled off and is recycled to the reaction,
5. the bottom product which is formed in the course of this procedure and which contains bisphenol, isomers and by-products is separated in a reactive rectification stage, in the presence of a catalytic amount of base, into phenol and an isoalkenylphenol which pass overhead, and into a high-boiling bottom product,
6. said high-boiling bottom product is acidified so that it contains a catalytic amount of acid, and is separated in a further reactive rectification stage into phenol and into a bottom product which is disposed of, and
7. the phenol distillate from 6. and the phenol/isoalkenylphenol distillate from 5. are each recycled, optionally after additional purification, to the reaction to form bisphenol.

## Revendications

1. Procédé de préparation en continu de dihydroxydiarylalcanes (bisphénols) à partir de cétone, de phénol et d'isoalcénylphénol par catalyse acide, **caractérisé en ce que**
1. on fait réagir dans une cascade d'au moins deux réacteurs, le phénol avec la cétone et l'isoalcénylphénol, de sorte que l'on travaille à la température la plus faible possible, mais à température croissante dans le sens de l'avancement de la réaction et on répartit l'acétone et l'isoalcénylphénol entre chaque réacteur, la répartition de la cétone et de l'isoalcénylphénol étant réglée de sorte que la part de la quantité totale par réacteur devient d'autant plus faible que la température du réacteur concerné est plus élevée ;
2. on distille la cétone n'ayant pas réagi du mélange réactionnel et éventuellement, on enlève des constituants acides contenus dans le mélange réactionnel ;
3. on sépare largement le bisphénol par cristallisation du mélange réactionnel ;
4. on réunit les eaux mères ainsi obtenus avec du phénol de lavage, on distille le phénol et on le renvoie dans la réaction ;
5. on dissocie le fond contenant le bisphénol ainsi formé, les isomères et les produits secondaires en présence d'une quantité catalytique de base, dans une rectification réactive en phénol et isoalcénylphénol sortant en tête et un fond à haut point d'ébullition ;
6. on acidifie ce fond à haut point d'ébullition de sorte qu'il contienne une quantité catalytique d'acide, on le dissocie dans une autre rectification réactive en phénol et un fond à éliminer, et
7. on renvoie le distillat de phénol de 6. et le distillat de phénol/isoalcénylphénol de 5., chaque fois le cas échéant, après une purification supplémentaire, dans la réaction en bisphénol.
